# EUROPEAN PATENT SPECIFICATION

(11) **EP 0 868 175 B1**
(45) Date of publication and mention of the grant of the patent: **19.02.2003**
(21) Application number: 96944077.5
(22) Date of filing: 18.12.1996
(51) Int. Cl.: A61K 31/155, A61K 31/195, A61P 3/10

(54) **NITRIC OXIDE (NO) SYNTHASE INHIBITOR TO PREVENT TYPE II DIABETES**
INHIBITOR VON STICKSTOFFMONOXID (NO) SYNTHASE ZUR VERHÜTUNG VON TYP II DIABETES
NHIBITEUR DE L'OXYDE NITRIQUE (NO) SYNTHASE POUR LA PROPHYLAXIE DU DIABETE DE TYPE II

(30) Priority: 22.12.1995 GB 9526331
(43) Date of publication of application: 07.10.1998
(73) Proprietor: SmithKline Beecham plc, Brentford, Middlesex TW8 9GS (GB)
(72) Inventor: TURNER, Nicholas, Charles SmithKline Beecham, Welwyn Hertfordshire AL6 9AR (GB); PIERCY, Valerie SmithKline Beecham, Welwyn Hertfordshire AL6 9AR (GB)
(74) Representative: Rutter, Keith, Dr.
(86) International application number: EP9605871
(87) International publication number: WO97023204

(56) References cited:
- WO-A-96/16031
- FUNDAM CLIN PHARMACOL, vol. 7, 1993, pages 401-411, XP000670309 BERDEAUX A.: "nitric oxide: an ubiquitous messenger"
- AM J HEALTH SYST PHARM, vol. 52, no. 16, 15 August 1995, pages 1781-1792, XP000670328 FOOTE E.F.: "prevention and treatment of diabetic nephropathy"

## Description

This invention relates to a novel medicament for the prophylaxis of non-insulin dependant (NIDDM or Type II) diabetes, and in particular to the use of an NO synthase inhibitor, such as aminoguanidine, for the said prophylaxis.

Hydrazinecarboximidamide (hereinafter aminoguanidine) is a known compound (Journal of American Chemical Society, 57, 2730, (1935).

Aminoguanidine is known to be an inhibitor of protein glycation and is under evaluation in animal models for the treatment of diabetic complications (Diabetes 42, 221-232 1993; Diabetologia 35, 946-950)

Aminoguanidine is known to be an NO synthase inhibitor and to be useful for the treatment of disease states characterised by over production of NO (European J. of Pharmacology, 233, (1993), 119-125). The inhibition of nitric oxide formation is particularly considered to be linked to the known activity of aminoguanidine in the treatment of diabetic complications European Patent Application, publication number. Aminoguanidine is under evaluation in animal models for the treatment of diabetic complications (Diabetes 42:221-232 1993; Diabetologia 35:946-950).

To date there has been no indication that aminoguanidine or any other NO synthase inhibitor would have a beneficial effect on Type II diabetes itself. As indicated above the emphasis has been focused upon the complications of diabetes. We have now surprisingly discovered that aminoguanidine shows potential for use in the prophylaxis of Type II diabetes *per se.* In particular, aminoguanidine is indicated to delay or prevent the progression of non-insulin dependent diabetes from hyperinsulinaemia to overt diabetes. This novel and surprising effect is considered to be due to the NO synthase inhibitor activity of aminoguanidine on the Type II diabetic pancreas.

Accordingly, the present invention provides a medicament for the prophylaxis of Type II diabetes, which comprises the administration, to a human or non-human mammal, of an effective non-toxic pharmaceutically acceptable amount of an NO synthase inhibitor or a pharmaceutically acceptable derivative thereof.

Preferably, the invention provides a medicament for the prophylactic treatment of Type II diabetes, in particular delaying or preventing the progression from hyperinsulinaemia to hyperglycaemia.

Suitable, inhibitors of NO synthase include proteins and non-protein compounds, such as aminoguanidine, n-monomethylarginine, or other analogues of 1-arginine.

A particular NO synthase inhibitor is aminoguanidine.

When used herein the term 'NO synthase inhibitor' refers to an agent that inhibits the formation of nitric oxide from l-arginine.

The NO synthase inhibitor activity of a compound is assessed in conventional tests such as inhibition of [³H] 1-arginine to [³H] to citrulline or inhibition of nitric oxide generation by cells or tissue extracts or by recombinant nitric oxide synthase isoenzymes *in vitro*.(FASEB.J 1993;7:349-360.

A suitable pharmaceutically acceptable derivative is a pharmaceutically acceptable salt, or a pharmaceutically acceptable solvate thereof.

Suitable pharmaceutically acceptable salts include acid addition salts.

Suitable acid addition salts include pharmaceutically acceptable inorganic salts such as the sulphate, nitrate, phosphate, borate, hydrochloride and hydrobromide and pharmaceutically acceptable organic acid addition salts such as acetate, tartrate, maleate, citrate, succinate, benzoate, ascorbate, methane-sulphonate, a-keto glutarate and a-glycerophosphate, especially the maleate salt.

Suitable pharmaceutically acceptable solvates include hydrates.

The NO synthase inhibitors used in the invention may be prepared according to conventional methods such as the methods disclosed in the above mentioned publications for example aminoguanidine may be prepared according to the methods disclosed in J. Amer. Chem. Soc. 57,2730, (1935).

Salts and/or solvates may be prepared and isolated according to conventional procedures.

In a further aspect the present invention also provides an NO synthase inhibitor, such as aminoguanidine, or a pharmaceutically acceptable derivative thereof, for use in the prophylaxis of Type II diabetes.

There is also provided an NO synthase inhibitor, such as aminoguanidine, or a pharmaceutically acceptable derivative thereof, for use in the manufacture of a medicament for the prophylaxis of Type II diabetes.

The NO synthase inhibitor, such as aminoguanidine, or a pharmaceutically acceptable derivative thereof may be administered per se or preferably as a pharmaceutical composition also comprising a pharmaceutically acceptable carrier.

### EXAMPLE

### Methodology of dbdb mouse model

The obese db/db mouse is a genetic model of type 2 non-insulin dependent diabetes which is both insulin resistant and hyperglycaemic. Male animals were obtained at 6 weeks of age. Blood samples were taken by tail tip snip for measurement of pre-treatment blood glucose. Animals were allocated into treated and control groups such that the mean and standard deviation of the fasting blood glucose concentrations of each group was similar.

On day 0 of the study a group of obese animals and their lean litter mates were killed for measurement of baseline biochemistry and histology. In addition one group of animals (control; n = 14) were fed a standard diet and a further group received aminoguanidine (500mg/kg; n = 14) in the same diet. Animals were allowed free access to food and water and their intake measured daily. At weekly intervals 24hr urine output was also measured. Mice (n = 7) were killed at 30 days and 85 days from commencement of treatment. Blood was taken for measurement of glucose and insulin concentrations and the pancreas removed for histological analysis and for measurement of pancreatic insulin.

### Data from dbdb mouse model

Food intake and body weight gain of the control and treated groups was similar throughout the experimental period.

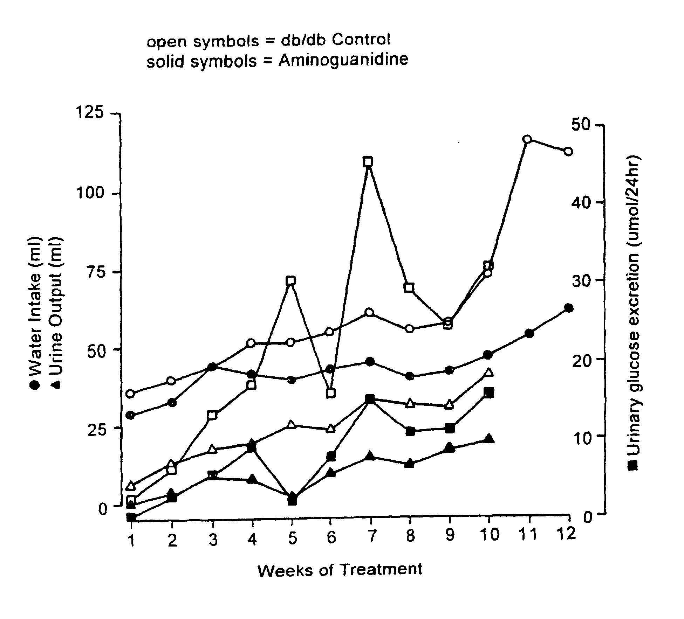

Immediately prior to dosing obese animals were normoglycaemic (blood glucose 10.4 ± 0.97mM) but were hyperinsulinaemic compared to their lean litter mates (serum isulin 127 ± 37 ng/ml in obese animals 3.05 ± 1.03 ng/ml in leans). By day 30 of the dosing period the obese control group were hyperglycaemic (blood glucose 24.9 ± 1.0 mM) and had markedly lower serum insulin levels (30.75 ± 4.3 mM) compared to the pre-treatment values. By day 85 of the treatment period, fasting blood glucose had risen to 28.1 ± 2 mM and serum insulin concentrations had fallen further, to 11.7 ± 1.8 ng/ml. Aminoguanidine attenuated the fall in fasting insulin concentrations (58.3 ± 13 ng/ml on day 30, 23.3 ± 4.1 ng/ml on day 85) and on day 85 had significantly reduced the prevailing fasting hyperglycaemia (21 ± 1.7 mM). Pancreatic insulin content of the aminoguanidine treated group of obese animals was twice that of the untreated animals (64.3 ± 17.8 ng/mg pancreas compared to 30.0 ± 2.6 ng/mg, respectively). From day 63 of the experimental period obese control animals were markedly polydypsic and polyuric compared to day 7. This increase in water intake and urine output is a characteristic of diabetes (hyperglycaemia) and was prevented by treatment with aminoguanidine (Figure 1). Similarly urinary glucose excretion increased steadily over the experimental period, in both untreated and treated animals, but from day 35 was lower in the aminoguanidine treated group (Figure 1). The development of diabetes (hyperglycaemia) was associated with changes in islet morphology, and the islets of untreated control animals were markedly hypertrophic, disorganised and had irregular boundaries. Islet insulin content was markedly depleted. On day 30 of the treatment period normal islet morphology was preserved in the aminoguanidine treated animals, and islet insulin content was greater.

## Claims

1. The use of an NO synthase inhibitor, or a pharmaceutically acceptable derivative thereof, for the manufacture of a medicament for the prophylactic treatment of Type II diabetes, providing that the NO synthase inhibitor does not include a compound of formulae (A) or (B): 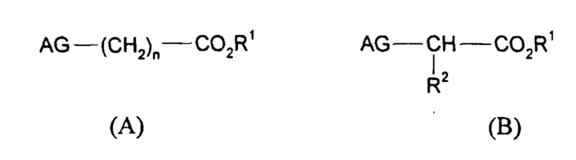 or a pharmacologically acceptable salt thereof, wherein AG is a) N-aminoguanidine, b) N,N'-diaminoguanidine, or c) N,N',N"-triaminoguanidine; wherein n is an integer from 1-5; wherein R¹ is a) hydrogen, b) phenyl, c) C₁-C₅ alkyl, or d) C₁-C₃ alkyl-phenyl; and wherein R² is a) hydrogen, b) phenyl, c) C₁-C₁₀ alkyl, or d) C₁-C₅ alkyl-phenyl which has NO synthase inhibitor activity.

2. A use according to claim 1, for delaying or preventing the progression from hyperinsulinaemia to hyperglycaemia.

3. A use according to claim 1 or claim 2, wherein the NO synthase inhibitor is selected from aminoguanidine, n-monomethylarginine; or other analogues of 1-arginine.

4. A use according to any one of claims 1 to 3, wherein the NO synthase inhibitor is aminoguanidine.

## Patentansprüche

1. Verwendung eines NO-Synthasehemmers, oder eines pharmazeutisch verträglichen Derivats davon, zur Herstellung eines Medikaments zur prophylaktischen Behandlung von Diabetes Typ II, mit der Maßgabe, dass der NO-Synthasehemmer weder eine Verbindung der Pormel (A) oder (B): 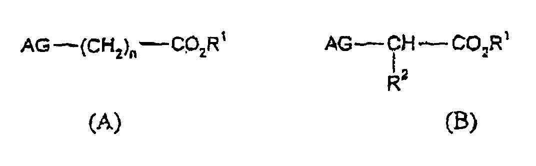 noch ein pharmakologisch verträgliches Salz davon, wobei AG a) N-Aminoguanidin, b) N,N'-Diaminoguanidin oder c) N, N',N"-Triaminoguanidin ist; wobei n eine ganze Zahl von 1 bis 5 ist, wobei R¹ a) Wasserstoff b) Phenyl, c) C₁-C₅-Alkyl oder d) C₁-C₃-Alkylphenyl ist und wobei R² a) Wasserstoff, b) Phenyl, c) C₁-C₁₀-Alkyl oder d) C₁-C₅-Alkylphenyl ist, die eine NO-Sythasehemmeraktivität beinhaltet.

2. Verwendinig gemäß Anspzuch 1, zur Verzögerung oder Verhinderung des Fortschreitens von Hyperinsulinämie zu Hyperglykämie.

3. Verwendung gemäß Anspruch 1 oder Anspruch 2, wobei der NO-Synthasehemmer aus Aminoguanidin, n-Monomethylarginin oder anderen Analoga von 1-Arginin ausgewählt ist.

4. Verwendung gemäß einem der Ansprüche 1 bis 3, wobei der NO-Synthasehemmer Aminoguanidin ist.

## Revendications

1. Utilisation d'un inhibiteur de NO-synthase, ou d'un de ses dérivés pharmaceutiquement acceptables, pour la production d'un médicament destiné au traitement prophylactique du diabète de type II, sous réserve que l'inhibiteur de NO-synthase ne comprenne pas un composé de formule (A) ou (B) : 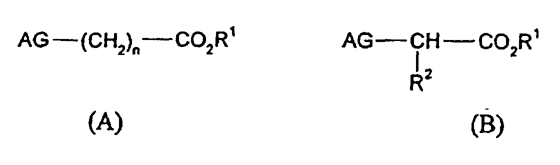 ou un de ses sels pharmacologiquement acceptables, formule dans laquelle AG représente a) la N-aminoguanidine, b) la N,N'-diaminoguanidine ou c) la N,N',N"-triaminoguanidine ; n représente un nombre entier de 1 à 5 ; R¹ représente a) l'hydrogène, b) un groupe phényle, c) un groupe alkyle en C₁ à C₅ ou d) un groupe (alkyle en C₁ à C₃)phényle ; et dans laquelle R² représente a) l'hydrogène, b), un groupe phényle, c) un groupe alkyle en C₁ à C₁₀ ou d) un groupe (alkyle en C₁ à C₅)phényle, qui a une activité d'inhibiteur de NO-synthase.

2. Utilisation suivant la revendication 1, pour retarder ou prévenir la progression de l'hyperinsulinémie en hyperglycémie.

3. Utilisation suivant la revendication 1 ou la revendication 2, dans laquelle l'inhibiteur de NO-synthase est choisi entre l'aminoguanidine, la n-monométhylarginine et d'autres analogues de 1-arginine.

4. Utilisation suivant l'une quelconque des revendications 1 à 3, dans laquelle l'inhibiteur de NO-synthase est l'aminoguanidine.
